# EUROPÄISCHE PATENTSCHRIFT

(11) **EP 3 466 869 B1**
(45) Veröffentlichungstag und Bekanntmachung des Hinweises auf die Patenterteilung: **08.07.2020**
(21) Anmeldenummer: 17195544.6
(22) Anmeldetag: 09.10.2017
(51) Int. Cl.: C01B 3/02, C01B 3/12, C01B 3/50, C07C 29/151

(54) **VERFAHREN ZUR KOMBINIERTEN HERSTELLUNG VON METHANOL UND VON AMMONIAK**
METHOD FOR THE COMBINED PREPARATION OF METHANOL AND AMMONIA
PROCÉDÉ DE FABRICATION COMBINÉE DE MÉTHANOL ET D'AMMONIAC

(43) Veröffentlichungstag der Anmeldung: 10.04.2019
(73) Patentinhaber: GasConTec AG, 4052 Basel (CH); thyssenkrupp Industrial Solutions AG, 45143 Essen (DE)
(72) Erfinder: SCHULZ, Alexander, 60439 Frankfurt (DE); VÖLKL, Johannes, 65779 Kelkheim (DE); KUNZ, Sina, 65993 Frankfurt (DE); MÜLLER, Dierk, 61184 Karben (DE); KOSS, Ulrich, 61348 Bad Homburg von der Höhe (DE)
(74) Vertreter: Patentanwälte Bauer Vorberg Kayser

(56) Entgegenhaltungen:
- WO-A1-2005/095313
- DE-A1- 3 712 008
- DE-A1-102007 008 690

## Beschreibung

Die Erfindung betrifft ein Verfahren zur kombinierten Herstellung von Methanol und von Ammoniak mit den Merkmalen des Oberbegriffs von Anspruch 1 sowie eine Anlage zur kombinierten Herstellung von Methanol und von Ammoniak mit den Merkmalen des Oberbegriffs von Anspruch 15.

Aus dem Stand der Technik sind verschiedene Ansätze zur kombinierten Herstellung von Methanol und von Ammoniak bekannt, siehe z. B. WO 2005/095313 A1. Die Offenlegungsschrift DE 10 2004 013 539A1 beschreibt ein Verfahren zur Koproduktion von Methanol und Ammoniak aus Erdgas. Aus dem Erdgas wird ein Synthesegas durch katalytische partielle Oxidation gewonnen und jeweils prozesstechnisch parallel angeordneten jeweiligen Reaktoranordnungen für die Methanolsynthese und die Ammoniaksynthese zugeführt. Speziell erfolgt dem Synthesegasreaktor nachgelagert eine Wassergas-Shift-Reaktion zur teilweisen Umwandlung von Kohlenmonoxid in Kohlenstoffdioxid und Wasserstoff und im Anschluss daran das im Wesentlichen vollständige Auswaschen des Kohlenstoffdioxids in einem Absorber. Das resultierende Gasgemisch wird dann in zwei Ströme aufgeteilt und jeweils einer Methanol- und einer Ammoniaksynthese zugeführt. Nachteilig an diesem Stand der Technik ist, dass durch die Aufteilung der Ströme im Absorber hier eine Zusammensetzung des Gasgemisches erreicht werden muss, welche jedenfalls als Ausgangsgas gleichermaßen für die Methanol- und für die Ammoniaksynthese geeignet sein muss. Abgesehen durch die Zufuhr eines Restgases aus einer Tieftemperaturzerlegung vor der Ammoniaksynthese zur Methanolsynthese gibt es bei diesem Verfahren keine Möglichkeit, eine Verschiebung der Gaszusammensetzung zwischen dem Ammoniak- und dem Methanolpfad zu bewirken. Das führt dazu, dass hinsichtlich der Gaszusammensetzungen sehr enge Randbedingungen anzulegen sind.

Ausgehend von diesem Stand der Technik besteht die Aufgabe der Erfindung folglich darin, bei der kombinierten Herstellung von Methanol und von Ammoniak durch eine Kopplung zwischen dem Methanol- und dem Ammoniakpfad die Möglichkeit der flexibleren Einstellung von Prozessparametern zu schaffen.

Bezogen auf ein Verfahren zur kombinierten Herstellung von Methanol und von Ammoniak mit den Merkmalen des Oberbegriffs von Anspruch 1 wird diese Aufgabe durch die Merkmale des kennzeichnenden Teils von Anspruch 1 gelöst. Bezogen auf eine Anlage zur kombinierten Herstellung von Methanol und von Ammoniak wird diese Aufgabe durch die Merkmale des kennzeichnenden Teils von Anspruch 15 gelöst.

Der Erfindung liegt die Erkenntnis zugrunde, dass der Schritt zur Erhöhung der Wasserstoffausbeute sowie zur effektiven Entfernung der Kohlenstoffoxide bezüglich der Ammoniaksynthese durch Shiftkonvertierung und anschließender Wasserstoffrückgewinnung zumindest teilweise auch auf einen Restgasstrom der Methanol-Reaktoranordnung angewandt werden kann. Denn auf diese Weise wird erstens der bei der Methanolsynthese wasserstoffintensive Kohlenstoffdioxid dem Methanolsynthese-Kreislauf entzogen, da die Wasserstoffrückgewinnung mit einer zumindest teilweisen Entfernung des Kohlenstoffdioxids einhergeht, sodass der Prozess insgesamt weniger Wasserstoff verbraucht. Zweitens wird der bei der Shiftkonvertierung zusätzlich gewonnene Wasserstoff wieder der Methanolsynthese zugeführt, was ebenfalls die Stöchiometrie verbessert. Da die entsprechenden Ab- und Zuführungen auch auf einfache Art und Weise dynamisch variabel eingestellt werden können, ergeben sich auf diese Art und Weise zusätzliche Freiheitsgrade zur besseren Einstellung der Prozesse.

Bei dem erfindungsgemäßen Verfahren zur kombinierten Herstellung von Methanol und von Ammoniak wird ein Eduktstrom mit Kohlenstoffmonoxid einer Rückgewinnungsanordnung zum Erhalten eines ersten wasserstoffhaltigen Stroms und eines zweiten wasserstoffhaltigen Stroms jeweils mit einem gegenüber dem Eduktstrom erhöhten molaren Anteil an Wasserstoff zugeführt, wobei die Rückgewinnungsanordnung eine Shiftkonvertierung aufweist, in welcher Shiftkonvertierung Kohlenstoffmonoxid mindestens eines kohlenstoffmonoxidhaltigen Stroms zumindest teilweise durch Reaktion mit Wasserdampf zu Wasserstoff und Kohlenstoffdioxid umgewandelt wird, sodass ein konvertierter Strom mit Wasserstoff und Kohlenstoffdioxid erhalten wird, welcher konvertierte Strom zumindest teilweise einer Wasserstoffrückgewinnung zugeführt wird, aus welcher Wasserstoffrückgewinnung der erste wasserstoffhaltige Strom und der zweite wasserstoffhaltige Strom gewonnen wird, wobei ein Stickstoffstrom und zumindest teilweise der erste wasserstoffhaltige Strom einer Ammoniak-Reaktoranordnung zur zumindest teilweisen Umwandlung in Ammoniak zugeführt wird und wobei zumindest teilweise der zweite wasserstoffhaltige Strom einer Methanol-Reaktoranordnung zur zumindest teilweisen Umwandlung in Methanol zugeführt wird. Der Eduktstrom kann neben dem Kohlenstoffmonoxid noch weitere Bestandteile wie etwa Wasserstoff und Kohlenstoffdioxid aufweisen. Ebenso kann der konvertierte Strom neben Wasserstoff und Kohlenstoffdioxid noch weitere Bestandteile aufweisen. Der erste wasserstoffhaltige Strom kann in seiner Zusammensetzung dem zweiten wasserstoffhaltigen Strom entsprechen. Die beiden wasserstoffhaltigen Ströme können aber auch eine jeweils unterschiedliche Zusammensetzung und auch einen jeweils unterschiedlichen molaren Anteil an Wasserstoff aufweisen.

Das erfindungsgemäße Verfahren ist dadurch gekennzeichnet, dass der Eduktstrom einen Restgasstrom mit unreagierten Kohlenstoffoxiden aus der Methanol-Reaktoranordnung umfasst. Bei diesem Restgasstrom handelt es sich also um einen Gasstrom, welcher prozesstechnisch vorgelagert bereits einen von der Methanol-Reaktoranordnung umfassten Methanolreaktor zur Synthese von Methanol durchlaufen hat und welcher Gasstrom Kohlenstoffoxide aufweist, die in dem Methanolreaktor nicht in Methanol umgewandelt wurden. Bevorzugt ist es, dass der Restgasstrom in seinem Massenstrom einstellbar aus der Methanol-Reaktoranordnung abgezweigt wird. Eine solche Einstellung kann es auch erlauben, das Verhältnis zwischen hergestelltem Methanol und hergestelltem Ammoniak zu variieren. Dabei kann es weiter sein, dass der Restgasstrom nach Austritt aus dem Methanolreaktor einer Zwischenbehandlung, z. B. einer Kondensation, unterzogen wurde. Der Eduktstrom kann neben dem Restgasstrom noch weitere Gasströme umfassen oder alternativ aus dem Restgasstrom bestehen. Es kann sein, dass die Erhöhung des molaren Anteils an Wasserstoff eine Erhöhung des molaren Anteils an Wasserstoff bezogen auf den Wasserstoff und die Kohlenstoffoxide bedeutet. Folglich würde eine Erhöhung des molaren Anteils an Wasserstoff auch dann erfolgen, wenn Kohlenstoffoxide entfernt werden aber durch Zufuhr eines weiteren Stoffs ohne Kohlenstoffoxide der molare Anteil des Wasserstoffs an dem Strom insgesamt gleichbleibt oder sogar sinkt. Die Ammoniak-Reaktoranordnung weist vorzugsweise einen Ammoniaksynthesekompressor auf, welcher zur Druckerhöhung des Stickstoffstroms und des der Ammoniak-Reaktoranordnung zugeführten ersten wasserstoffhaltigen Stroms eingerichtet ist.

Grundsätzlich kann der Eduktstrom prozesstechnisch an beliebiger Stelle der Rückgewinnungsanordnung zugeführt werden. Bevorzugt ist, dass der Eduktstrom der Shiftkonvertierung zugeführt wird. Dies kann auch dadurch ausgedrückt werden, dass der mindestens eine kohlenstoffmonoxidhaltige Strom den Eduktstrom umfasst oder aus diesem besteht. Es kann aber auch sein, dass der Eduktstrom insbesondere nur der Wasserstoffrückgewinnung zugeführt wird. Dabei kann der Eduktstrom entweder der Wasserstoffrückgewinnung prozesstechnisch vorgelagert mit dem konvertierten Strom zusammengeführt werden oder separat zum konvertierten Strom der Wasserstoffrückgewinnung zugeführt werden. Schließlich kann es auch sein, dass ein Teil des Eduktstroms der Shiftkonvertierung und ein weiterer Teil des Eduktstroms nur der Wasserstoffrückgewinnung zugeführt wird, wobei diese Teile des Eduktstroms hinsichtlich ihrer absoluten oder relativen Stoffmenge auch einstellbar sein können.

Grundsätzlich kann die Shiftkonvertierung aus einer einzigen Konvertierungsstufe zur Shiftkonvertierung bestehen. Unter dem Begriff der Shiftkonvertierung wird hier stets ein Reaktor für die an sich aus dem Stand der Technik bekannte Wassergas-Shift-Reaktion verstanden, bei welcher Kohlenstoffmonoxid und Wasserdampf zu Kohlenstoffdioxid und Wasserstoff umgewandelt werden. Eine bevorzugte Ausführungsform des Verfahrens ist dadurch gekennzeichnet, dass die Shiftkonvertierung eine erste Konvertierungsstufe zur Shiftkonvertierung und eine der ersten Konvertierungsstufe prozesstechnisch nachgelagerte zweite Konvertierungsstufe zur Shiftkonvertierung aufweist und dass die Shiftkonvertierung in der ersten und der zweiten Konvertierungsstufe bei einer unterschiedlichen Temperatur abläuft, sodass das chemische Gleichgewicht in der ersten Konvertierungsstufe unterschiedlich zu dem chemischen Gleichgewicht in der zweiten Konvertierungsstufe ist. Auf diese Weise kann der Ablauf der Wassergas-Shift-Reaktion sowohl in einem Hochtemperatur- als auch in einem Niedertemperaturbereich vorgesehen sein, wodurch sich eine hinreichend niedrige Konzentration von Kohlenstoffmonoxid innerhalb einer akzeptablen Zeit erreichen lässt. Es kann sein, dass der Eduktstrom der ersten Konvertierungsstufe zugeführt wird.

Es ist ebenso aus dem Stand der Technik bekannt, dass das chemische Gleichgewicht und die Kinetik der Wassergas-Shift-Reaktion temperaturabhängig sind. Folglich unterscheidet sich die Kinetik in der ersten Konvertierungsstufe von der Kinetik in der zweiten Konvertierungsstufe, Gemäß dieser Ausführungsform kann die Shiftkonvertierung auch mehr als zwei Konvertierungsstufen aufweisen.

Eine weitere bevorzugte Ausführungsform des Verfahrens ist dadurch gekennzeichnet, dass ein weiterer Eduktstrom mit Kohlenstoffmonoxid der Rückgewinnungsanordnung zugeführt wird. Insbesondere kann es sein, dass der weitere Eduktstrom der Shiftkonvertierung zur zumindest teilweisen Umwandlung zu Wasserstoff und Kohlenstoffdioxid durch Reaktion mit Wasserdampf zugeführt wird. Dann umfasst der mindestens eine kohlenstoffmonoxidhaltige Strom den weiteren Eduktstrom oder besteht aus ihm. Auf diese Weise kann die Einstellbarkeit des Gesamtprozesses noch weiter erhöht werden. Dieser weitere Eduktstrom kann innerhalb der Shiftkonvertierung mit dem erstgenannten Eduktstrom zusammengeführt werden. Im Falle mehrerer Konvertierungsstufen kann der weitere Eduktstrom prozesstechnisch an einer Stelle der Shiftkonvertierung zugeführt werden, welche sich von der Zuführung des Eduktstroms unterscheidet.

Speziell ist es hier bevorzugt, dass der Eduktstrom der Shiftkonvertierung der ersten Konvertierungsstufe prozesstechnisch nachgelagert und der zweiten Konvertierungsstufe prozesstechnisch vorgelagert zugeführt wird. Dann ist es bevorzugt, dass der weitere Eduktstrom der ersten Konvertierungsstufe zugeführt wird. Auf diese Weise durchlaufen der Eduktstrom und der weitere Eduktstrom jeweils unterschiedlich viele Konvertierungsstufen der Shiftkonvertierung. Da der Eduktstrom den Restgasstrom aus der Methanolsynthese umfasst, kann dieser häufig einen niedrigeren Anteil an Kohlenstoffmonoxid als der weitere Eduktstrom aufweisen. In so einem Fall kann eine vorgeschaltete Shiftkonvertierung im Hochtemperaturbereich zwar für den weiteren Eduktstrom zweckmäßig, für den Eduktstrom mit dem Restgas aber entbehrlich sein.

Gemäß einer bevorzugten Ausführungsform des Verfahrens ist vorgesehen, dass mindestens 80 %, vorzugsweise mindestens 85 % und insbesondere mindestens 90 %, des molaren Anteils von Kohlenstoffmonoxid des Eduktstroms, insbesondere auch des weiteren Eduktstroms, in der Shiftkonvertierung durch Reaktion mit Wasserdampf zu Wasserstoff und Kohlenstoffdioxid umgewandelt wird.

Eine bevorzugte Ausführungsform des Verfahrens ist dadurch gekennzeichnet, dass die Shiftkonvertierung eine Niedertemperatur-Shiftkonvertierungsstufe umfasst, in welcher im Wesentlichen ausschließlich eine Niedertemperatur-Wassergas-Shift-Reaktion abläuft. Bevorzugt ist, dass diese Wassergas-Shift-Reaktion bei weniger als 300° C abläuft. Es kann insbesondere, dass die zweite Konvertierungsstufe die Niedertemperatur-Shiftkonvertierungsstufe ist. Bei niedriger Temperatur ist zwar die Kinetik verlangsamt, doch liegt das chemische Gleichgewicht bei einer niedrigeren Konzentration von Kohlenstoffmonoxid. Folglich kann durch die Niedertemperatur-Shiftreaktion eine weitgehende Umwandlung von noch verbliebenen Mengen von Rest-Kohlenstoffmonoxid in dem Eduktstrom erreicht werden.

Eine weitere bevorzugte Ausführungsform des Verfahrens ist dadurch gekennzeichnet, dass die Shiftkonvertierung eine Hochtemperatur-Shiftkonvertierungsstufe umfasst, in welcher im Wesentlichen ausschließlich eine Hochtemperatur-Wassergas-Shift-Reaktion - vorzugsweise bei mindestens 300° C - abläuft. Es kann insbesondere sein, dass die erste Konvertierungsstufe die Hochtemperatur-Shiftkonvertierungsstufe ist. Durch die bessere Kinetik bei höheren Temperaturen kann in dieser ersten Stufe eine rasche Verringerung der Kohlenstoffmonoxid-Konzentration auf einen geringeren Pegel erreicht werden, insbesondere wenn diese Konzentration zunächst eher hoch ist.

Gemäß einer bevorzugten Ausführungsform des Verfahrens ist vorgesehen, dass der Shiftkonvertierung prozesstechnisch vorgelagert ein Synthesegasstrom mit Wasserstoff und Kohlenstoffoxiden der Methanol-Reaktoranordnung zur zumindest teilweisen Umwandlung in Methanol zugeführt wird, sodass die unreagierten Kohlenstoffoxide aus dem Synthesegasstrom stammen. Mit der prozesstechnischen Vorlagerung gegenüber der Shiftkonvertierung ist gemeint, dass dieser Synthesegasstrom bei Zuführung zur Methanol-Reaktoranordnung noch nicht die Shiftkonvertierung durchlaufen hat. Häufig ist es bei frischem Synthesegas nicht erforderlich oder sinnvoll, bereits vor der Methanolsynthese eine Shiftkonvertierung durchzuführen. Denn es kann bezweckt sein, möglichst viel Kohlenstoffmonoxid für eine erste Reaktorstufe der Methanolsynthese zur Verfügung zu haben. Vorzugsweise weist die Methanol-Reaktoranordnung einen Synthesegaskompressor zur Druckerhöhung des Synthesegasstroms auf.

Hinsichtlich des zweiten wasserstoffhaltigen Stroms aus der Wasserstoffrückgewinnung ist es bevorzugt, dass er mit dem Synthesegasstrom zusammengeführt wird. Dies erlaubt die Einstellung der gewünschten Stöchiometriezahl. Insbesondere kann es sein, dass er dem Synthesegasstrom dem Synthesegaskompressor prozesstechnisch vorgelagert zugeführt wird.

In diesem Zusammenhang ist es weiter bevorzugt, dass die Methanol-Reaktoranordnung eine Kondensationsstufe zur Abtrennung von Methanol und zum Erhalten des Restgasstroms umfasst. Neben dem Methanol kann in der Kondensationsstufe auch Wasser abgetrennt werden. Ein solcher Restgasstrom weist nach Abtrennung des Methanols regelmäßig einen gegenüber dem der Methanol-Reaktoranordnung zugeführten Strom deutlich verringerten Anteil an Kohlenstoffmonoxid auf. Ferner kann neben dem Restgasstrom auch ein, vorzugsweise in seiner Zusammensetzung dem Restgasstrom entsprechender, Recyclestrom aus der Kondensationsstufe erhalten werden, welcher insbesondere zur zumindest teilweisen Umwandlung in Methanol im Kreislauf gefahren wird.

Eine bevorzugte Ausführungsform des Verfahren ist dadurch gekennzeichnet, dass die Methanol-Reaktoranordnung eine erste und eine zweite Reaktorstufe zur Synthese von Methanol aufweist, dass der Synthesegasstrom der ersten Reaktorstufe zugeführt wird und dass ein weiterer Restgasstrom mit unreagierten Kohlenstoffoxiden aus der ersten Reaktorstufe der zweiten Reaktorstufe zugeführt wird. Folglich wird nicht das gesamte Restgas aus der Methanolsynthese insgesamt und auch nicht das gesamte Restgas aus der ersten Reaktorstufe der Shiftkonvertierung zugeführt. Bevorzugt ist, dass die Methanol-Reaktoranordnung einen Zwischenkompressor zur Druckerhöhung des weiteren Restgasstroms vor Zuführung des weiteren Restgasstroms zur zweiten Reaktorstufe aufweist.

Hier ist es weiter bevorzugt, dass ein Massenstrom des Restgasstroms einstellbar ist. Alternativ oder zusätzlich kann es sein, dass ein Restgas der ersten Reaktorstufe variabel in den Restgasstrom und den weiteren Restgasstrom aufgeteilt wird. Bezüglich der obigen Kondensationsstufe ist es bevorzugt, dass die Kondensationsstufe prozesstechnisch zwischen der ersten und der zweiten Reaktorstufe angeordnet ist und dass aus der Kondensationsstufe der Restgasstrom und der weitere Restgasstrom sowie ggf. auch der Recyclestrom gewonnen wird.

Weiter kann es sein, dass der zweiten Reaktorstufe prozesstechnisch nachgelagert eine weitere Kondensationsstufe zur Abtrennung von Methanol und zum Erhalten des Recyclestroms umfasst. Vorzugsweise wird dann der Recyclestrom zur ersten Reaktorstufe zurückgeführt und somit im Kreislauf gefahren.

Grundsätzlich kann der oben genannte Synthesegasstrom auf beliebige Art und Weise gewonnen werden und einer beliebigen Quelle entspringen. Eine weitere bevorzugte Ausführungsform des Verfahrens ist speziell dadurch gekennzeichnet, dass ein kohlenstoffhaltiger Energieträgerstrom einer Synthesegasreaktoranordnung zum Gewinnen des Synthesegasstroms zugeführt wird. Hier ist es weiter bevorzugt, dass der weitere Eduktstrom ein aus der Synthesegasreaktoranordnung gewonnener weiterer Synthesegasstrom mit Wasserstoff und Kohlenstoffoxiden ist. In so einem Fall wird der Shiftkonvertierung nicht nur der Eduktstrom mit Restgas aus der Methanolsynthese zugeführt, sondern auch frisches Synthesegas. Auf diese Weise kann das Verhältnis von Wasserstoff gegenüber den Kohlenstoffoxiden weiter erhöht werden. Hier ist es bevorzugt, dass der weitere Synthesegasstrom in seinem Massenstrom einstellbar aus der Synthesegasreaktoranordnung abgezweigt wird. Auch durch diese Einstellung kann das Verhältnis zwischen hergestelltem Methanol und hergestelltem Ammoniak variiert werden.

Bezüglich des Synthesegasreaktors ist ebenfalls grundsätzlich eine Vielzahl von Varianten möglich. Eine hier bevorzugte Variante sieht vor, dass ein sauerstoffhaltiger Strom der Synthesegasreaktoranordnung zugeführt wird und dass in einem Reaktor der Synthesegasreaktoranordnung durch eine katalytische partielle Oxidation mittels des sauerstoffhaltigen Stroms Synthesegas für den Synthesegasstrom, insbesondere auch für den weiteren Synthesegasstrom, gewonnen wird. Dies bietet erstens den Vorteil, dass das Synthesegas gegenüber einer herkömmlichen Dampfreformierung mit einem höheren Druck bereitgestellt werden kann. Bevorzugt ist, dass der sauerstoffhaltige Strom überwiegend und insbesondere im Wesentlichen vollständig aus Sauerstoff besteht. Gegenüber der Verwendung von Umgebungsluft für die katalytische partielle Oxidation führt dies durch das weitgehende oder im Wesentlichen vollständige Entfallen von Stickstoff zu einem geringeren Volumen an Prozessgas, was wiederum eine kleinere und damit günstigere Dimensionierung der nachgelagerten Vorrichtungen wie beispielsweise der Kompressoren erlaubt. Alternativ oder zusätzlich zu der katalytischen partiellen Oxidation kann es sein, dass in einem ggf. weiteren Reaktor der Synthesegasreaktoranordnung durch eine Dampfreformierung Synthesegas für den Synthesegasstrom gewonnen wird.

Es kann sein, dass die Synthesegasreaktoranordnung einen der partiellen katalytischen Oxidation prozesstechnisch vorgeschalteten Pre-Reformer zum Aufspalten von Kohlenwasserstoffen mit mindestens zwei Kohlenwasserstoffen aufweist.

Gemäß einer bevorzugten Ausführungsform des Verfahrens ist vorgesehen, dass die Synthesegasreaktoranordnung eine prozesstechnisch dem Reaktor nachgeordnete mehrstufige Abhitzeverwertung zur Rückgewinnung der Wärme aus der Gewinnung des Synthesegasstroms aufweist und dass der Synthesegasstrom und der weitere Synthesegasstrom nach jeweils unterschiedlichen Stufen der Abhitzeverwertung gewonnen werden. Regelmäßig wird das Synthesegas aus einem Synthesegasreaktor bei einer Temperatur gewonnen, welche für die weitere Verarbeitung zu hoch ist. Aus diesem Grund kann eine Abhitzeverwertung vorgesehen sein, welche etwa einen Abhitzekessel zum Erzeugen von Prozessdampf sowie einen oder mehrere Wärmetauscher umfasst und somit sowohl das Synthesegas abkühlt als auch Prozessdampf erzeugt oder an einer prozesstechnisch anderen Stelle eine Erwärmung bietet. Vorzugsweise umfasst eine erste Stufe der Abhitzeverwertung den Abhitzekessel und eine nachgelagerte Stufe einen Wärmetauscher. Es kann sein, dass für die Methanolsynthese das Synthesegas auf eine niedrigere Temperatur abgekühlt werden soll als sie für die Shiftkonvertierung und insbesondere für eine Hochtemperatur-Shiftkonvertierungsstufe erforderlich ist.

Hier ist es bevorzugt, dass der weitere Synthesegasstrom dem Synthesegasstrom prozesstechnisch vorgelagert abgezweigt wird. Auf diese Weise weist der weitere Synthesegasstrom bei seiner Abzweigung eine höhere Temperatur als der Synthesegasstrom auf. Hier ist es bevorzugt, dass der weitere Synthesegasstrom in seinem Massenstrom einstellbar aus der Abhitzeverwertung abgezweigt wird. Dies erlaubt ebenfalls das Verhältnis zwischen hergestelltem Methanol und hergestelltem Ammoniak zu variieren.

Eine bevorzugte Ausführungsform des Verfahrens ist dadurch gekennzeichnet, dass der Stickstoffstrom aus einer Luftzerlegung zum Gewinnen von molekularem Sauerstoff aus der Umgebungsluft erlangt wird. Weiter ist es bevorzugt, dass der sauerstoffhaltige Strom ein aus der Luftzerlegung gewonnener, insbesondere im Wesentlichen aus Sauerstoff bestehender, Sauerstoffstrom ist. Auf diese Weise kann der bei der Luftzerlegung zum Erlangen des Stickstoffstroms anfallende Sauerstoff vorteilhaft für die Erzeugung des Synthesegases verwendet werden.

Eine weitere bevorzugte Ausführungsform des Verfahrens ist dadurch gekennzeichnet, dass ein Purgegas mit Kohlenstoffdioxid aus der Wasserstoffrückgewinnung erlangt wird. Vorzugsweise weist dieses Purgegas gegenüber dem konvertierten Strom einen erhöhten Anteil an Kohlenstoffdioxid auf. Es kann auch sein, dass dieses Purgegas im Wesentlichen aus Kohlenstoffdioxid besteht. Dieses Kohlenstoffdioxid kann ebenfalls einer Verwendung zugeführt werden, beispielsweise zur Herstellung von Harnstoff. Es kann sein, dass aus der Wasserstoffrückgewinnung mehr als ein Strom von Purgegas gewonnen wird, wobei es dann sein kann, dass das Kohlenstoffdioxid in einem der Ströme des Purgegases angereichert ist und in einem anderem Strom des Purgegases im Wesentlichen andere Bestandteile wie z. B. Edelgasreste angereichert sind. Dieser andere Strom kann dann etwa als Fuelgas zur Unterfeuerung in einem Ofen benutzt werden.

Grundsätzlich kann es sein, dass Reste von Kohlenstoffdioxid in dem ersten wasserstoffhaltigen Strom oder in dem zweiten wasserstoffhaltigen Strom verbleiben. Es ist jedoch bevorzugt, dass in der Wasserstoffrückgewinnung im Wesentlichen das gesamte Kohlenstoffdioxid des konvertierten Stroms entfernt wird, sodass der erste und/oder der zweite wasserstoffhaltige Strom im Wesentlichen frei von Kohlenstoffdioxid ist.

Grundsätzlich kann das obige Entfernen des Kohlenstoffdioxids aus dem konvertierten Strom auf beliebige Art und Weise erfolgen. Eine weitere bevorzugte Ausführungsform des Verfahrens ist dadurch gekennzeichnet, dass die Wasserstoffrückgewinnung eine Kohlenstoffdioxidwäsche zum zumindest teilweisen Auswaschen des Kohlenstoffdioxids mittels eines Waschmittels aus dem konvertierten Strom aufweist. Es kann sein, dass nur ein insbesondere einstellbarer Teil des konvertierten Stroms der Kohlenstoffdioxidwäsche zugeführt wird. Bevorzugt ist, dass das Purgegas zumindest teilweise aus der Kohlenstoffdioxidwäsche erlangt wird. Ebenso kann es sein, dass der zweite wasserstoffhaltige Strom aus der Kohlenstoffdioxidwäsche durch das Auswaschen des Kohlenstoffdioxids aus dem konvertierten Strom gewonnen wird. Da der zweite wasserstoffhaltige Strom der Methanolsynthese zugeführt wird, ist für diesen häufig eine geringere Reinheit erforderlich als für den zur Ammoniaksynthese zugeführten ersten wasserstoffhaltigen Strom.

Gemäß einer bevorzugten Ausführung des Verfahrens ist vorgesehen, dass die Wasserstoffrückgewinnung eine Druckwechsel-Adsorptionsanlage (PSA) zum Gewinnen des ersten wasserstoffhaltigen Stroms, vorzugsweise auch zum Gewinnen des zweiten wasserstoffhaltigen Stroms aufweist. Diese Druckwechsel-Adsorptionsanlage kann der Kohlenstoffdioxidwäsche prozesstechnisch nachgeordnet sein. Es kann sein, dass Druckwechsel-Adsorptionsanlage eine mehrstufige Druckwechsel-Adsorptionsvorrichtung aufweist. Es kann weiter sein, dass der erste wasserstoffhaltige Strom im Wesentlichen aus Wasserstoff besteht und kohlenstoffoxidfrei ist. Dies erlaubt, insbesondere bei der Zufuhr von reinem Stickstoff zur Ammoniaksynthese, eine kleinere Dimensionierung des Reaktors zur Ammoniaksynthese und aller Komponenten zum Recyclen. Ebenso kann es sein, dass der zweite wasserstoffhaltige Strom im Wesentlichen aus Wasserstoff besteht und im Wesentlichen kohlenstoffoxidfrei ist.

Ebenso kann es sein, dass die Wasserstoffrückgewinnung eine Vorrichtung zur Methanisierung des konvertierten Stroms aufweist. Speziell wird in dieser Methanisierung Kohlenstoffdioxid und Kohlenstoffmonoxid in Methan umgewandelt. Auch eine solche Methanisierung bedeutet in Kombination mit der Shiftkonvertierung eine Erhöhung des molaren Anteils an Wasserstoff, und zwar vor allem durch Verringerung des molaren Anteils von Kohlenstoffoxiden und der Bildung von zusätzlichem Wasserstoff in der Shiftkonvertierung. Auf diese Weise kann verhindert werden, dass Kohlenstoffoxide in die Ammoniak-Reaktoranordnung gelangen.

Die erfindungsgemäße Anlage dient der kombinierten Herstellung von Methanol und von Ammoniak und weist eine Rückgewinnungsanordnung auf, welcher ein Eduktstrom mit Kohlenstoffmonoxid zugeführt wird und aus welcher ein erster wasserstoffhaltiger Strom und ein zweiter wasserstoffhaltiger Strom jeweils mit einem gegenüber dem Eduktstrom erhöhten molaren Anteil an Wasserstoff gewonnen wird, wobei die Rückgewinnungsanordnung eine Shiftkonvertierung aufweist, in welcher Kohlenstoffmonoxid mindestens eines kohlenstoffmonoxidhaltigen Stroms zumindest teilweise durch Reaktion mit Wasserdampf zu Wasserstoff und Kohlenstoffdioxid umgewandelt wird, sodass ein konvertierter Strom mit Wasserstoff und Kohlenstoffdioxid erhalten wird, und eine Wasserstoffrückgewinnung aufweist, welcher der konvertierte Strom zumindest teilweise zugeführt wird und aus welcher der erste wasserstoffhaltiger Strom und der zweite wasserstoffhaltiger Strom gewonnen wird. Die erfindungsgemäße Anlage weist ebenso eine Ammoniak-Reaktoranordnung auf, welcher ein Stickstoffstrom und zumindest teilweise der erste wasserstoffhaltige Strom zur zumindest teilweisen Umwandlung in Ammoniak zugeführt wird und eine Methanol-Reaktoranordnung auf, welcher zumindest teilweise der zweite wasserstoffhaltige Strom zur zumindest teilweisen Umwandlung in Methanol zugeführt wird.

Die erfindungsgemäße Anlage ist dadurch gekennzeichnet, dass der Eduktstrom einen Restgasstrom mit unreagierten Kohlenstoffoxiden aus der Methanol-Reaktoranordnung umfasst.

Bevorzugte Ausgestaltungen und Merkmale der erfindungsgemäßen Anlage entsprechen bevorzugten Ausgestaltungen und Merkmalen des erfindungsgemäßen Verfahrens und umgekehrt.

Weitere Einzelheiten, Merkmale, Ziele und Vorteile der vorliegenden Erfindung werden nachfolgend anhand einer lediglich Ausführungsbeispiele wiedergebenden Zeichnung erläutert. In der Zeichnung zeigt
- Fig, 1: eine schematische Darstellung eines ersten Ausführungsbeispiels der vorschlagsgemäßen Anlage zur Ausführung des vorschlagsgemäßen Verfahrens,
- Fig. 2: eine schematische Darstellung eines zweiten Ausführungsbeispiels der vorschlagsgemäßen Anlage zur Ausführung des vorschlagsgemäßen Verfahrens,
- Fig. 3: eine schematische Darstellung eines dritten Ausführungsbeispiels der vorschlagsgemäßen Anlage zur Ausführung des vorschlagsgemäßen Verfahrens,
- Fig. 4: eine schematische Darstellung eines vierten Ausführungsbeispiels der vorschlagsgemäßen Anlage zur Ausführung des vorschlagsgemäßen Verfahrens,
- Fig. 5: eine schematische Darstellung eines fünften Ausführungsbeispiels der vorschlagsgemäßen Anlage zur Ausführung des vorschlagsgemäßen Verfahrens und
- Fig. 6: eine schematische Darstellung eines sechsten Ausführungsbeispiels der vorschlagsgemäßen Anlage zur Ausführung des vorschlagsgemäßen Verfahrens.

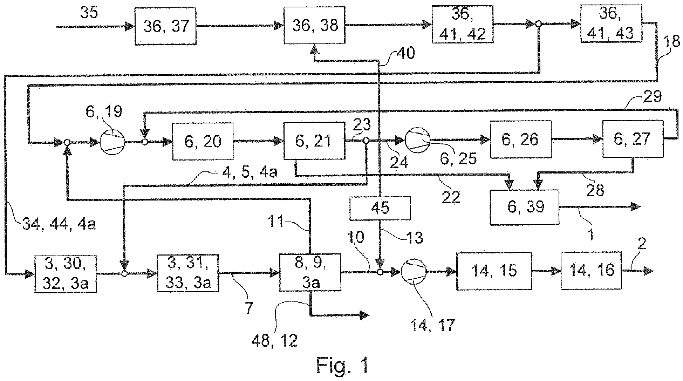

Die in der Fig. 1 gemäß einem ersten Ausführungsbeispiel dargestellte vorschlagsgemäße Anlage zur kombinierten Herstellung von Methanol 1 und von Ammoniak 2 weist eine Rückgewinnungsanordnung 3a mit einer Shiftkonvertierung 3 auf, welcher ein Eduktstrom 4 als ein kohlenstoffmonoxidhaltiger Strom 4a zugeführt wird. Bei dem Eduktstrom 4 handelt es sich um einen Restgasstrom 5 mit unreagierten Kohlenstoffoxiden aus einer Methanol-Reaktoranordnung 6 der vorschlagsgemäßen Anlage, welcher der Herstellung von Methanol 1 durch Synthese dient.

Aus der Shiftkonvertierung 3 wird ein konvertierter Strom 7 gewonnen, welcher Wasserstoff und Kohlenstoffdioxid aufweist. Speziell erfolgt durch die Wassergas-Shift-Reaktion in der Shiftkonvertierung 3 eine weitgehende Umwandlung des Kohlenmonoxids des Eduktstroms 4 mit Wasserdampf zu Wasserstoff und Kohlenstoffdioxid. Dieser konvertierte Strom 7 wird einer Wasserstoffrückgewinnung 8 der Rückgewinnungsanordnung 3a zugeführt, bei welcher es sich hier um eine Druckwechsel-Adsorptionsanlage 9 handelt. Aus dieser wird ein erster wasserstoffhaltiger Strom 10 und ein zweiter wasserstoffhaltiger Strom 11 gewonnen, welche hier beide im Wesentlichen aus Wasserstoff bestehen. Daneben wird ein Purgegas 12 bestehend aus einem Offgas 48 der Wasserstoffrückgewinnung 8 gewonnen, welches Purgegas 12 aus dem Reststrom des konvertierten Stroms 7 nach Abtrennung des ersten wasserstoffhaltigen Stroms 10 und des zweiten wasserstoffhaltigen Stroms 11 besteht.

Der erste wasserstoffhaltige Strom 10 wird mit einem Stickstoffstrom 13 zusammengeführt und mit diesem einer Ammoniak-Reaktoranordnung 14 zur Synthese von Ammoniak 2 zugeführt. Die Ammoniak-Reaktoranordnung 14 besteht aus einem Ammoniak-Reaktor 15, in weicher die Umwandlung von Wasserstoff und Stickstoff in Ammoniak 2 stattfindet, sowie einer prozesstechnisch nachgelagerten Trennstufe 16 zum Abtrennen des Ammoniaks 2 und einem prozesstechnisch vorgelagerten Ammoniaksynthesekompressor 17 zur Druckerhöhung.

Der zweite wasserstoffhaltige Strom 11 wird gemeinsam mit einem Synthesegasstrom 18 im Wesentlichen bestehend aus Wasserstoff und Kohlenstoffoxiden der Methanol-Reaktoranordnung 6 zugeführt. Diese weist einen Synthesegaskompressor 19 zur Druckerhöhung des Synthesegasstroms 18 und des mit ihm zusammengeführten zweiten wasserstoffhaltigen Stroms 11 auf. Dem Synthesegaskompressor 19 prozesstechnisch nachgelagert ist eine erste Reaktorstufe 20 zur Synthese von Methanol 1 der Methanol-Reaktoranordnung 6, an welche sich prozesstechnisch eine ebenfalls von der Methanol-Reaktoranordnung 6 umfasste Kondensationsstufe 21 zur Abtrennung von Methanol und von Wasser in einem Rohmethanolstrom 22 anschließt. Der abgetrennte Rohmethanolstrom 22 wird einer Destillationsstufe 39 der Methanol-Reaktoranordnung 6 zugeführt, aus welcher dann das Methanol 1 gewonnen wird. Es wäre ebenso denkbar, den zweiten wasserstoffhaltigen Strom 11 der Methanol-Reaktoranordnung 6 dem Synthesegaskompressor 19 prozesstechnisch nachgelagert zuzuführen.

Aus der Kondensationsstufe 21 wird ebenso ein Restgas 23 mit den unreagierten Gasbestandteilen aus der ersten Reaktorstufe 20 erhalten, welches Restgas 23 in den Restgasstrom 5 zur Zuführung zur Shiftkonvertierung 3 und in einen weiteren Restgasstrom 24 aufgeteilt wird, welcher weitere Restgasstrom 24 nach Druckerhöhung durch einen Zwischenkompressor 25 einer zweiten Reaktorstufe 26 zugeführt wird. Diese Aufteilung in den Restgasstrom 5 und in den weiteren Restgasstrom 24 ist variabel. Der zweiten Reaktorstufe 26 prozesstechnisch nachgelagert ist eine weitere Kondensationsstufe 27, aus welcher ein weiterer Rohmethanolstrom 28 gewonnen wird, welcher ebenfalls der Destillationsstufe 39 zugeführt wird, Restgas aus der weiteren Kondensationsstufe 27 wird als Recyclestrom 29 zur ersten Reaktorstufe 20 zurückgeführt. Der Zwischenkompressor 25, die zweite Reaktorstufe 26 und die weitere Kondensationsstufe 27 sind ebenfalls von der Methanol-Reaktoranordnung 6 umfasst.

Die Shiftkonvertierung 3 besteht erstens aus einer Hochtemperatur-Shiftkonvertierungsstufe 30, in welcher eine Hochtemperatur-Wassergas-Shift-Reaktion bei mindestens 300° C abläuft, und einer prozesstechnisch nachgelagerten Niedertemperatur-Shiftkonvertierungsstufe 31, in weicher eine Niedertemperatur-Wassergas-Shift-Reaktion bei weniger als 300° C abläuft. Es folgt, dass auch das jeweilige chemische Gleichgewicht unterschiedlich ist. Die Hochtemperatur-Shiftkonvertierungsstufe 30 ist folglich eine erste Konvertierungsstufe 32 zur Shiftkonvertierung und die Niedertemperatur-Shiftkonvertierungsstufe 31 eine prozesstechnisch der ersten Konvertierungsstufe 32 nachgelagerte zweite Konvertierungsstufe 33 zur Shiftkonvertierung. Der Eduktstrom 4 wird dabei der Shiftkonvertierung 3 prozesstechnisch zwischen der Hochtemperatur-Shiftkonvertierungsstufe 30 und der Niedertemperatur-Shiftkonvertierungsstufe 31 zugeführt, sodass der Eduktstrom 4 lediglich die Niedertemperatur-Shiftkonvertierungsstufe 31 durchläuft.

Ein weiterer Eduktstrom 34 wird als weiterer kohlenstoffmonoxidhaltiger Strom 4a ebenfalls der Shiftkonvertierung 3 zugeführt und hier speziell der Hochtemperatur-Shiftkonvertierungsstufe 30, sodass dieser anschließend auch die Niedertemperatur-Shiftkonvertierungsstufe 31 durchläuft.

Dieser weitere Eduktstrom 34 entstammt der nachfolgend beschriebenen Anordnung zur Bereitstellung von Synthesegas, welche auch den Synthesegasstrom 18 bereitstellt. Und zwar wird ein kohlenstoffhaltiger Energieträgerstrom 35, bei welchem es sich vorliegend um einen Erdgasstrom handelt, einer Synthesegasreaktoranordnung 36 zugeführt. Speziell wird der kohlenstoffhaltige Energieträgerstrom 35 zunächst einem Pre-Reformer 37 der Synthesegasreaktoranordnung 36 zugeführt, in welchem Pre-Reformer 37 höhere Kohlenwasserstoffe aufgespaltet werden. Anschließend erfolgt eine Zuführung zu einem Reaktor 38 der Synthesegasreaktoranordnung 36 für eine katalytische partielle Oxidation. Für diese katalytische partielle Oxidation wird dem Reaktor 38 ein sauerstoffhaltiger Strom 40 zugeführt, bei welchem es sich hier um einen im Wesentlichen aus Sauerstoff bestehenden Strom handelt.

Dem Reaktor 38 prozesstechnisch nachgeordnet ist eine hier zweistufige Abhitzeverwertung 41 der Synthesegasreaktoranordnung 36, durch welche einerseits eine Abkühlung des Synthesegases und andererseits dabei eine Rückgewinnung der Hitze erfolgen soll. Die Abhitzeverwertung 41 besteht aus einem Abhitzekessel 42 zur Erzeugung von Prozessdampf und einer prozesstechnisch dem Abhitzekessel 42 nachgelagerten Wärmetauscher-Anordnung 43 mit mehreren einzelnen Wärmetauschern. Der weitere Eduktstrom 34 wird als weiterer Synthesegasstrom 44 prozesstechnisch nach dem Abhitzekessel 42 abgezweigt, wohingegen der Synthesegasstrom 18 nach Durchlaufen der Wärmetauscher-Anordnung 43 gewonnen wird. Die Menge des als weiteren Synthesegasstrom 44 abgezweigten Synthesegases ist einstellbar. Durch diese vorgelagerte Abzweigung weist der weitere Synthesegasstrom 44 eine höhere Temperatur als der Synthesegasstrom 18 auf, was im Lichte seiner Zuführung zur Hochtemperatur-Shiftkonvertierungsstufe 30 vorteilhaft ist.

Der sauerstoffhaltige Strom 40 entstammt einer Luftzerlegung 45, weiche auch den Stickstoffstrom 13 bereitstellt.

Im ersten Ausführungsbeispiel weist der Energieträgerstrom 35 einen Massenstrom von 175 t/h, das Synthesegas aus dem Reaktor 38 einen Massenstrom vom 630 t/h, der weitere Eduktstrom 34 einen Massenstrom von 190 t/h, der Synthesegasstrom 18 einen Massenstrom vom 440 t/h, das Methanol 1 einen Massenstrom von 210 t/h und das Ammoniak 2 einen Massenstrom von 70 t/h auf. Es ist zu beachten, dass insbesondere dem Reaktor 38 prozesstechnisch vorgelagert eine Zuführung von Wasser und Wasserdampf erfolgt.

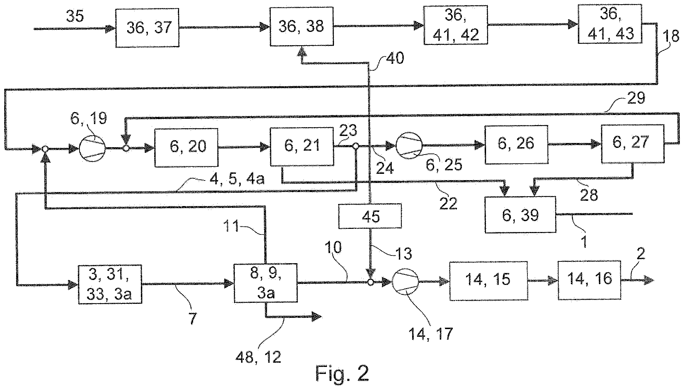

Für die weiteren Ausführungsbeispiele der Fig. 2 bis 6 werden lediglich die Unterschiede zum ersten Ausführungsbeispiel bzw. zu einem anderen Ausführungsbeispiel beschrieben. Soweit also nicht anders festgestellt, entsprechen sie dem jeweiligen als Ausgangspunkt dienenden Ausführungsbeispiel.

Das zweite Ausführungsbeispiel der Fig. 2 unterscheidet sich von dem ersten Ausführungsbeispiel darin, dass auf den weiteren Eduktstrom 44 verzichtet wird. Entsprechend ist die Shiftkonvertierung 3 einstufig und weist lediglich eine Niedertemperatur-Shiftkonvertierungsstufe 31 auf.

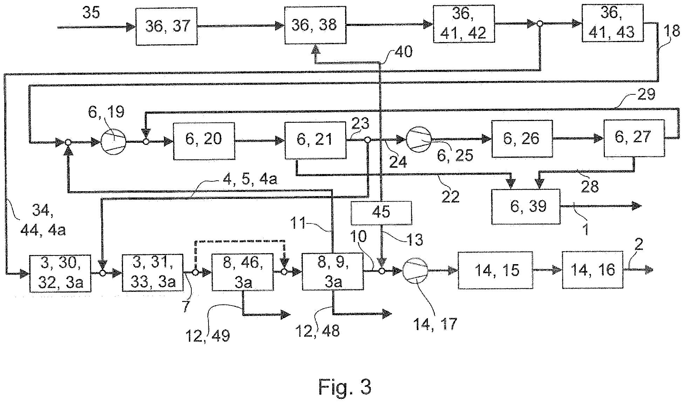

Das dritte Ausführungsbeispiel der Fig. 3 geht wieder von dem ersten Ausführungsbeispiel aus. Hier weist jedoch die Wasserstoffrückgewinnung 8 zusätzlich eine Kohlenstoffdioxidwäsche 46 auf, welcher ein variabler Teil des konvertierten Stroms 7 zugeführt wird. Aus diesem Teil wird das Kohlenstoffdioxid im Wesentlichen vollständig durch ein Waschmittel ausgewaschen und in einem im Wesentlichen aus Kohlenstoffdioxid bestehenden CO2-Strom 49 gewonnen. Der vorbeigeleitete Teil des konvertierten Stroms 7 wie auch der konvertierte Strom 7 nach dem Auswaschen wird der Druckwechsel-Adsorptionsanlage 9 zugeführt, wobei durch den bereits verringerten molaren Anteil an Kohlenstoffdioxid die Belastung der Druckwechsel-Adsorptionsanlage 9 verringert wird. Das Purgegas 12 umfasst hier sowohl das Offgas 48 aus der Druckwechsel-Adsorptionsanlage 9 als auch den CO2-Strom 49 aus der Kohlenstoffdioxidwäsche 46, wobei dieser einer gesonderten Weiterverarbeitung zugeführt werden kann.

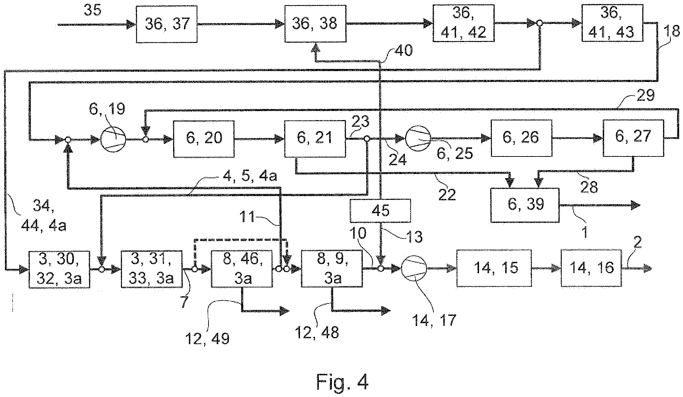

Das vierte Ausführungsbeispiel der Fig. 4 geht von dem dritten Ausführungsbeispiel der Fig. 3 aus und unterscheidet sich darin, dass der zweite wasserstoffhaltige Strom 11 dadurch gewonnen wird, dass er prozesstechnisch der Kohlenstoffdioxidwäsche 46 nachgelagert abgezweigt wird. Abweichend von den Ausführungsbeispielen 1 bis 3 handelt es sich dann nicht um einen im Wesentlichen aus Wasserstoff bestehenden Strom, sondern lediglich um einen durch das Auswaschen des Kohlenstoffdioxids in seinem molaren Anteil mit Wasserstoff angereicherten Strom. Da insbesondere verbliebenes Kohlenstoffmonoxid für die Methanolsynthese verwendbar ist, ist dies aber unschädlich. Der erste wasserstoffhaltige Strom 10 wird nach wie vor aus der Druckwechsel-Adsorptionsanlage 9 gewonnen und besteht im Wesentlichen aus Wasserstoff.

Im vierten Ausführungsbeispiel weist der Energieträgerstrom 35 einen Massenstrom von 225 t/h, das Synthesegas aus dem Reaktor 38 einen Massenstrom vom 780 t/h, der weitere Eduktstrom 34 einen Massenstrom von 290 t/h, der Synthesegasstrom 18 einen Massenstrom vom 490 t/h, der CO2-Strom 49 einen Massenstrom von 230 t/h, das Methanol 1 einen Massenstrom von 210 t/h und das Ammoniak 2 einen Massenstrom von 145 t/h auf.

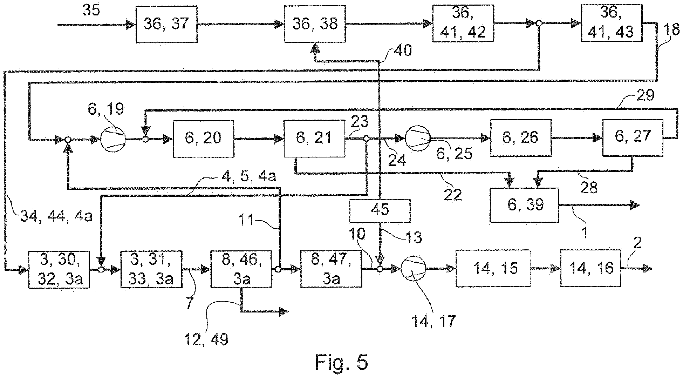

Das fünfte Ausführungsbeispiel der Fig. 5 geht von dem vierten Ausführungsbeispiel aus und unterscheidet sich erstens darin, dass der gesamte konvertierte Strom 7 der Kohlenstoffdioxidwäsche 46 zugeführt wird. Zweitens weist die Wasserstoffrückgewinnung 8 anstelle der Druckwechsel-Adsorptionsanlage 9 eine Vorrichtung zur Methanisierung 47 des konvertierten Stroms 7 auf, aus welcher der erste wasserstoffhaltige Strom 10 gewonnen wird. Hier besteht das Purgegas 12 aus dem CO2-Strom 49.

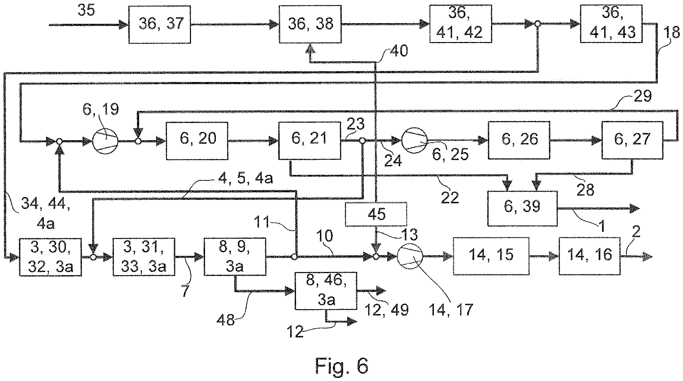

Schließlich geht das sechste Ausführungsbeispiel der Fig. 6 wieder von dem ersten Ausführungsbeispiel aus und unterscheidet sich dadurch, dass hier die Wasserstoffrückgewinnung 8 eine Kohlenstoffdioxidwäsche 46 aufweist, welcher das Offgas 48 aus der der Druckwechsel-Adsorptionsanlage 9 zugeführt wird. Aus der Kohlenstoffdioxidwäsche 46 wird das Purgegas 12 gewonnen, welches neben dem Purgestrom im engeren Sinne auch den CO2-Strom 49 umfasst.

## Patentansprüche

1. Verfahren zur kombinierten Herstellung von Methanol (1) und von Ammoniak (2), wobei ein Eduktstrom (4) mit Kohlenstoffmonoxid einer Rückgewinnungsanordnung (3a) zum Erhalten eines ersten wasserstoffhaltigen Stroms (10) und eines zweiten wasserstoffhaltigen Stroms (11) jeweils mit einem gegenüber dem Eduktstrom (4) erhöhten molaren Anteil an Wasserstoff zugeführt wird, wobei die Rückgewinnungsanordnung (3a) eine Shiftkonvertierung (3) aufweist, in welcher Kohlenstoffmonoxid mindestens eines kohlenstoffmonoxidhaltigen Stroms (4a) zumindest teilweise durch Reaktion mit Wasserdampf zu Wasserstoff und Kohlenstoffdioxid umgewandelt wird, sodass ein konvertierter Strom (7) mit Wasserstoff und Kohlenstoffdioxid erhalten wird, welcher zumindest teilweise einer Wasserstoffrückgewinnung (8) zugeführt wird, aus der der erste wasserstoffhaltiger Strom (10) und der zweite wasserstoffhaltiger Strom (11) gewonnen wird, wobei ein Stickstoffstrom (13) und zumindest teilweise der erste wasserstoffhaltige Strom (10) einer Ammoniak-Reaktoranordnung (14) zur zumindest teilweisen Umwandlung in Ammoniak (2) zugeführt wird und wobei zumindest teilweise der zweite wasserstoffhaltige Strom (11) einer Methanol-Reaktoranordnung (6) zur zumindest teilweisen Umwandlung in Methanol (1) zugeführt wird, **dadurch gekennzeichnet, dass** der Eduktstrom (4) einen Restgasstrom (5) mit unreagierten Kohlenstoffoxiden aus der Methanol-Reaktoranordnung (6) umfasst.

2. Verfahren nach Anspruch 1, **dadurch gekennzeichnet, dass** die Shiftkonvertierung (3) eine erste Konvertierungsstufe (32) zur Shiftkonvertierung und eine der ersten Konvertierungsstufe (32) prozesstechnisch nachgelagerte zweite Konvertierungsstufe (33) zur Shiftkonvertierung aufweist und dass die Shiftkonvertierung (3) in der ersten Konvertierungsstufe (32) und der zweiten Konvertierungsstufe (33) bei einer unterschiedlichen Temperatur abläuft, sodass das chemische Gleichgewicht in der ersten Konvertierungsstufe (32) unterschiedlich zu dem chemischen Gleichgewicht in der zweiten Konvertierungsstufe (33) ist.

3. Verfahren nach Anspruch 2, **dadurch gekennzeichnet, dass** ein weiterer Eduktstrom (34) mit Kohlenstoffmonoxid der Rückgewinnungsanordnung (3a) zugeführt wird, insbesondere der Shiftkonvertierung (3) zur zumindest teilweisen Umwandlung zu Wasserstoff und Kohlenstoffdioxid durch Reaktion mit Wasserdampf zugeführt wird, vorzugsweise, dass der Eduktstrom (4) der Shiftkonvertierung (3) der ersten Konvertierungsstufe (32) prozesstechnisch nachgelagert und der zweiten Konvertierungsstufe (33) prozesstechnisch vorgelagert zugeführt wird, insbesondere, dass der weitere Eduktstrom (34) der ersten Konvertierungsstufe (32) zugeführt wird.

4. Verfahren nach einem der Ansprüche 1 bis 3, **dadurch gekennzeichnet, dass** mindestens 80 %, vorzugsweise mindestens 85 %, insbesondere mindestens 90 %, des molaren Anteils von Kohlenstoffmonoxid des Eduktstroms (4), insbesondere auch des weiteren Eduktstroms (34), in der Shiftkonvertierung (3) durch Reaktion mit Wasserdampf zu Wasserstoff und Kohlenstoffdioxid umgewandelt wird.

5. Verfahren nach einem der Ansprüche 1 bis 4, **dadurch gekennzeichnet, dass** die Shiftkonvertierung (3) eine Niedertemperatur-Shiftkonvertierungsstufe (31) umfasst, in welcher im Wesentlichen ausschließlich eine Niedertemperatur-Wassergas-Shift-Reaktion, vorzugsweise bei weniger als 300° C, abläuft, insbesondere, dass die zweite Konvertierungsstufe (33) die Niedertemperatur-Shiftkonvertierungsstufe (31) ist.

6. Verfahren nach einem der Ansprüche 1 bis 5, **dadurch gekennzeichnet, dass** die Shiftkonvertierung (3) eine Hochtemperatur-Shiftkonvertierungsstufe (30) umfasst, in welcher im Wesentlichen ausschließlich eine Hochtemperatur-Wassergas-Shift-Reaktion, vorzugsweise bei mindestens 300° C, abläuft, insbesondere, dass die erste Konvertierungsstufe (32) die Hochtemperatur-Shiftkonvertierungsstufe (30) ist.

7. Verfahren nach einem der Ansprüche 1 bis 6, **dadurch gekennzeichnet, dass** der Shiftkonvertierung (3) prozesstechnisch vorgelagert ein Synthesegasstrom (18) mit Wasserstoff und Kohlenstoffoxiden der Methanol-Reaktoranordnung (6) zur zumindest teilweisen Umwandlung in Methanol (1) zugeführt wird, sodass die unreagierten Kohlenstoffoxide aus dem Synthesegasstrom (18) stammen, vorzugsweise, dass die Methanol-Reaktoranordnung (6) eine Kondensationsstufe (21) zur Abtrennung von Methanol (1) und zum Erhalten des Restgasstroms (5) umfasst.

8. Verfahren nach Anspruch 7, **dadurch gekennzeichnet, dass** die Methanol-Reaktoranordnung (6) eine erste Reaktorstufe (20) und eine zweite Reaktorstufe (26) zur Synthese von Methanol (1) aufweist, dass der Synthesegasstrom (18) der ersten Reaktorstufe (20) zugeführt wird und dass ein weiterer Restgasstrom (24) mit unreagierten Kohlenstoffoxiden aus der ersten Reaktorstufe (20) der zweiten Reaktorstufe (26) zugeführt wird, vorzugsweise, dass die Kondensationsstufe (21) prozesstechnisch zwischen der ersten Reaktorstufe (20) und der zweiten Reaktorstufe (26) angeordnet ist und dass aus der Kondensationsstufe (21) der Restgasstrom (5) und der weitere Restgasstrom (24) gewonnen wird.

9. Verfahren nach Anspruch 7 oder 8, **dadurch gekennzeichnet, dass** ein kohlenstoffhaltiger Energieträgerstrom (35) einer Synthesegasreaktoranordnung (36) zum Gewinnen des Synthesegasstroms (18) zugeführt wird, vorzugsweise, dass der weitere Eduktstrom (34) ein aus der Synthesegasreaktoranordnung (36) gewonnener weiterer Synthesegasstrom (44) mit Wasserstoff und Kohlenstoffoxiden ist, vorzugsweise, dass ein sauerstoffhaltiger Strom (40) der Synthesegasreaktoranordnung (36) zugeführt wird und dass in einem Reaktor (38) der Synthesegasreaktoranordnung (36) durch eine katalytische partielle Oxidation mittels des sauerstoffhaltigen Stroms (40) Synthesegas für den Synthesegasstrom (18), insbesondere auch für den weiteren Synthesegasstrom (44), gewonnen wird.

10. Verfahren nach Anspruch 9, **dadurch gekennzeichnet, dass** die Synthesegasreaktoranordnung (36) eine prozesstechnisch dem Reaktor (38) nachgeordnete mehrstufige Abhitzeverwertung (41) zur Rückgewinnung der Wärme aus der Gewinnung des Synthesegases aufweist und dass der Synthesegasstrom (18) und der weitere Synthesegasstrom (44) nach jeweils unterschiedlichen Stufen der Abhitzeverwertung (41) gewonnen werden, vorzugsweise, dass der weitere Synthesegasstrom (44) dem Synthesegasstrom (18) prozesstechnisch vorgelagert abgezweigt wird.

11. Verfahren nach einem der Ansprüche 1 bis 10, **dadurch gekennzeichnet, dass** der Stickstoffstrom (13) aus einer Luftzerlegung (45) zum Gewinnen von molekularem Sauerstoff aus der Umgebungsluft erlangt wird, vorzugsweise, dass der sauerstoffhaltige Strom (40) ein aus der Luftzerlegung (45) gewonnener, insbesondere im Wesentlichen aus Sauerstoff bestehender, Sauerstoffstrom ist.

12. Verfahren nach einem der Ansprüche 1 bis 11, **dadurch gekennzeichnet, dass** Purgegas (12) mit Kohlenstoffdioxid aus der Wasserstoffrückgewinnung (8) erlangt wird, vorzugsweise, dass in der Wasserstoffrückgewinnung (8) im Wesentlichen das gesamte Kohlenstoffdioxid des konvertierten Stroms (7) entfernt wird, sodass der erste wasserstoffhaltige Strom (10) und/oder der zweite wasserstoffhaltige Strom (11) im Wesentlichen frei von Kohlenstoffdioxid ist.

13. Verfahren nach Anspruch 12, **dadurch gekennzeichnet, dass** die Wasserstoffrückgewinnung (8) eine Kohlenstoffdioxidwäsche (46) zum zumindest teilweisen Auswaschen des Kohlenstoffdioxids mittels eines Waschmittels aus dem konvertierten Strom (7) aufweist, vorzugsweise, dass das Purgegas (12) zumindest teilweise aus der Kohlenstoffdioxidwäsche (46) erlangt wird, insbesondere, dass der zweite wasserstoffhaltige Strom (11) aus der Kohlenstoffdioxidwäsche (46) durch das Auswaschen des Kohlenstoffdioxids aus dem konvertierten Strom (7) gewonnen wird.

14. Verfahren nach einem der Ansprüche 1 bis 13, **dadurch gekennzeichnet, dass** die Wasserstoffrückgewinnung (8) eine, insbesondere der Kohlenstoffdioxidwäsche (46) prozesstechnisch nachgeordnete, Druckwechsel-Adsorptionsanlage (9) zum Gewinnen des ersten wasserstoffhaltigen Stroms (10), vorzugsweise, auch zum Gewinnen des zweiten wasserstoffhaltigen Stroms (11) aufweist, weiter vorzugsweise, dass der erste wasserstoffhaltige Strom (10) im Wesentlichen aus Wasserstoff besteht und im Wesentlichen kohlenstoffoxidfrei ist, weiter insbesondere dass der zweite wasserstoffhaltige Strom (11) im Wesentlichen aus Wasserstoff besteht und im Wesentlichen kohlenstoffoxidfrei ist.

15. Anlage zur kombinierten Herstellung von Methanol (1) und von Ammoniak (2) mit einer Rückgewinnungsanordnung (3a), welcher ein Eduktstrom (4) mit Kohlenstoffmonoxid zugeführt wird und aus welcher ein erster wasserstoffhaltiger Strom (10) und ein zweiter wasserstoffhaltiger Strom (11) jeweils mit einem gegenüber dem Eduktstrom (4) erhöhten molaren Anteil an Wasserstoff gewonnen wird, wobei die Rückgewinnungsanordnung (3a) eine Shiftkonvertierung (3) aufweist, in welcher Kohlenstoffmonoxid mindestens eines kohlenstoffmonoxidhaltigen Stroms (4a) zumindest teilweise durch Reaktion mit Wasserdampf zu Wasserstoff und Kohlenstoffdioxid umgewandelt wird, sodass ein konvertierter Strom (7) mit Wasserstoff und Kohlenstoffdioxid erhalten wird, wobei die Rückgewinnungsanordnung (3a) eine Wasserstoffrückgewinnung (8) aufweist, welcher der konvertierte Strom (7) zumindest teilweise zugeführt wird und aus welcher der erste wasserstoffhaltiger Strom (10) und der zweite wasserstoffhaltiger Strom (11) gewonnen wird, mit einer Ammoniak-Reaktoranordnung (14), welcher ein Stickstoffstrom (13) und zumindest teilweise der erste wasserstoffhaltige Strom (10) zur zumindest teilweisen Umwandlung in Ammoniak (2) zugeführt wird und mit einer Methanol-Reaktoranordnung (6), welcher zumindest teilweise der zweite wasserstoffhaltige Strom (11) zur zumindest teilweisen Umwandlung in Methanol (1) zugeführt wird, **dadurch gekennzeichnet, dass** der Eduktstrom (4) einen Restgasstrom (5) mit unreagierten Kohlenstoffoxiden aus der Methanol-Reaktoranordnung (6) umfasst.

## Claims

1. A process for combined production of methanol (1) and ammonia (2), wherein a reactant stream (4) comprising carbon monoxide is supplied to a recovery assembly (3a) to obtain a first hydrogen-containing stream (10) and a second hydrogen-containing stream (11), each having an increased molar proportion of hydrogen compared to the reactant stream (4), wherein the recovery assembly (3a) comprises a shift conversion (3) in which carbon monoxide of at least one carbon monoxide-containing stream (4a) is at least partially converted into hydrogen and carbon dioxide by reaction with steam to obtain a converted stream (7) comprising hydrogen and carbon dioxide, which is at least partially supplied to a hydrogen recovery (8) from which the first hydrogen-containing stream (10) and the second hydrogen-containing stream (11) are obtained, wherein a nitrogen stream (13) and, at least partially, the first hydrogen-containing stream (10) are supplied to an ammonia reactor assembly (14) for at least partial conversion into ammonia (2) and wherein, at least partially, the second hydrogen-containing stream (11) is supplied to a methanol reactor assembly (6) for at least partial conversion into methanol (1), **characterised in that** the reactant stream (4) comprises a residual gas stream (5) comprising unreacted carbon oxides from the methanol reactor assembly (6).

2. The process according to claim 1, **characterised in that** the shift conversion (3) comprises a first conversion stage (32) for shift conversion and a second conversion stage (33) for shift conversion, which is downstream of the first conversion stage (32) in the process sequence, and **in that** the shift conversion (3) in the first conversion stage (32) and the second conversion stage (33) takes place at a different temperature, and therefore the chemical equilibrium in the first conversion stage (32) is different from the chemical equilibrium in the second conversion stage (33).

3. The process according to claim 2, **characterised in that** a further reactant stream (34) comprising carbon monoxide is supplied to the recovery assembly (3a), in particular to the shift conversion (3) for at least partial conversion into hydrogen and carbon dioxide by reaction with steam, preferably **in that** the reactant stream (4) is supplied to the shift conversion (3) downstream of the first conversion stage (32) in the process sequence and upstream of the second conversion stage (33) in the process sequence, in particular **in that** the further reactant stream (34) is supplied to the first conversion stage (32).

4. The process according to any one of claims 1 to 3, **characterised in that** at least 80%, preferably at least 85%, in particular at least 90%, of the molar proportion of carbon monoxide of the reactant stream (4), in particular also of the further reactant stream (34), is converted into hydrogen and carbon dioxide in the shift conversion (3) by reaction with steam.

5. The process according to any one of claims 1 to 4, **characterised in that** the shift conversion (3) comprises a low-temperature shift conversion stage (31), in which substantially exclusively a low-temperature water-gas shift reaction takes place, preferably at less than 300°C, in particular **in that** the second conversion stage (33) is the low-temperature shift conversion stage (31).

6. The process according to any one of claims 1 to 5, **characterised in that** the shift conversion (3) comprises a high-temperature shift conversion stage (30), in which substantially exclusively a high-temperature water-gas shift reaction takes place, preferably at least at 300°C, in particular **in that** the first conversion stage (32) is the high-temperature shift conversion stage (30).

7. The process according to any one of claims 1 to 6, **characterised in that** a synthesis gas stream (18) comprising hydrogen and carbon oxides is supplied to the methanol reactor assembly (6) upstream of the shift conversion (3) in the process sequence for at least partial conversion into methanol (1), so that the unreacted carbon oxides originate from the synthesis gas stream (18), preferably **in that** the methanol reactor assembly (6) comprises a condensation stage (21) for separating methanol (1) and for obtaining the residual gas stream (5).

8. The process according to claim 7, **characterised in that** the methanol reactor assembly (6) comprises a first reactor stage (20) and a second reactor stage (26) for the synthesis of methanol (1), **in that** the synthesis gas stream (18) is supplied to the first reactor stage (20), and **in that** a further residual gas stream (24) with unreacted carbon oxides from the first reactor stage (20) is supplied to the second reactor stage (26), preferably **in that** the condensation stage (21) is arranged between the first reactor stage (20) and the second reactor stage (26) in the process sequence, and **in that** the residual gas stream (5) and the further residual gas stream (24) is obtained from the condensation stage (21).

9. The process according to claim 7 or 8, **characterised in that** a carbon-containing energy carrier stream (35) is supplied to a synthesis gas reactor assembly (36) for obtaining the synthesis gas stream (18), preferably **in that** the further reactant stream (34) is a further synthesis gas stream (44), comprising hydrogen and carbon oxides, obtained from the synthesis gas reactor assembly (36), preferably **in that** an oxygen-containing stream (40) is supplied to the synthesis gas reactor assembly (36), and **in that** synthesis gas for the synthesis gas stream (18), in particular also for the further synthesis gas stream (44), is obtained in a reactor (38) of the synthesis gas reactor assembly (36) by catalytic partial oxidation by means of the oxygen-containing stream (40).

10. The process according to claim 9, **characterised in that** the synthesis gas reactor assembly (36) comprises a multi-stage waste heat utilisation (41), arranged downstream of the reactor (38) in the process sequence, for recovering the heat from the recovery of the synthesis gas, and **in that** the synthesis gas stream (18) and the further synthesis gas stream (44) are obtained in each case after different stages of the waste heat utilisation (41), preferably **in that** the further synthesis gas stream (44) is branched off from the synthesis gas stream (18) upstream in respect of the process sequence.

11. The process according to any one of claims 1 to 10, **characterised in that** the nitrogen stream (13) is obtained from an air separation (45) for obtaining molecular oxygen from the ambient air, preferably **in that** the oxygen-containing stream (40) is an oxygen stream obtained from the air separation (45), in particular consisting substantially of oxygen.

12. The process according to any one of claims 1 to 11, **characterised in that** purge gas (12) comprising carbon dioxide is obtained from the hydrogen recovery (8), preferably **in that** substantially all carbon dioxide of the converted stream (7) is removed in the hydrogen recovery (8) so that the first hydrogen-containing stream (10) and/or the second hydrogen-containing stream (11) is substantially free of carbon dioxide.

13. The process according to claim 12, **characterised in that** the hydrogen recovery (8) comprises a carbon dioxide scrubbing (46) for at least partially washing the carbon dioxide out of the converted stream (7) by means of a washing agent, preferably **in that** the purge gas (12) is at least partially obtained from the carbon dioxide wash (46), in particular **in that** the second hydrogen-containing stream (11) is obtained from the carbon dioxide scrubber (46) by washing the carbon dioxide out of the converted stream (7).

14. The process according to any one of claims 1 to 13, **characterised in that** the hydrogen recovery (8) has a pressure swing adsorption plant (9), in particular one which is arranged downstream of the carbon dioxide scrubber (46) in the process sequence, for recovering the first hydrogen-containing stream (10), preferably also for recovering the second hydrogen-containing stream (11), further preferably **in that** the first hydrogen-containing stream (10) consists substantially of hydrogen and is substantially free of carbon oxides, further preferably in that the second hydrogen-containing stream (11) consists substantially of hydrogen and is substantially free of carbon oxides.

15. A plant for combined production of methanol (1) and of ammonia (2), with a recovery assembly (3a), to which a reactant stream (4) comprising carbon monoxide is supplied and from which a first hydrogen-containing stream (10) and a second hydrogen-containing stream (11) are obtained, each with an increased molar proportion of hydrogen compared with the reactant stream (4), wherein the recovery assembly (3a) comprises a shift conversion (3) in which carbon monoxide of at least one carbon monoxide-containing stream (4a) is at least partially converted into hydrogen and carbon dioxide by reaction with steam so as to obtain a converted stream (7) comprising hydrogen and carbon dioxide, wherein the recovery assembly (3a) comprises a hydrogen recovery (8), to which the converted stream (7) is at least partially supplied and from which the first hydrogen-containing stream (10) and the second hydrogen-containing stream (11) are recovered, with an ammonia reactor assembly (14), to which a nitrogen stream (13) and at least partially the first hydrogen-containing stream (10) is supplied for at least partial conversion into ammonia (2), and with a methanol reactor assembly (6), to which, at least partially, the second hydrogen-containing stream (11) is supplied for at least partial conversion into methanol (1), **characterised in that** the reactant stream (4) comprises a residual gas stream (5) comprising unreacted carbon oxides from the methanol reactor assembly (6).

## Revendications

1. Procédé de production combinée de méthanol (1) et d'ammoniac (2), dans lequel un courant d'éduit (4) comprenant du monoxyde de carbone est amené à un ensemble de récupération (3a) de manière à obtenir un premier courant contenant de l'hydrogène (10) et un deuxième courant contenant de l'hydrogène (11), ayant chacun une fraction molaire d'hydrogène accrue par rapport au courant d'éduit (4), dans lequel ledit ensemble de récupération (3a) présente une conversion catalytique (3) dans laquelle du monoxyde de carbone d'au moins un courant contenant du monoxyde de carbone (4a) est converti, au moins en partie, par réaction avec de la vapeur d'eau, en hydrogène et en dioxyde de carbone de sorte que l'on obtienne un courant converti (7) comprenant de l'hydrogène et du dioxyde de carbone, qui est amené au moins en partie à une récupération d'hydrogène (8) à partir de laquelle sont obtenus le premier courant contenant de l'hydrogène (11) et le deuxième courant contenant de l'hydrogène (11), dans lequel un courant d'azote (13) et au moins en partie le premier courant contenant de l'hydrogène (10) sont amenés à un ensemble de réacteur d'ammoniac (14) pour les convertir au moins en partie en ammoniac (2), et dans lequel au moins en partie le deuxième courant contenant de l'hydrogène (11) est amené à un ensemble de réacteur de méthanol (6) pour le convertir au moins en partie en méthanol (1), **caractérisé par le fait que** le courant d'éduit (4) comprend un courant de gaz résiduel (5) comprenant des oxydes de carbone issus de l'ensemble de réacteur de méthanol (6), qui n'ont pas réagi.

2. Procédé selon la revendication 1, **caractérisé par le fait que** la conversion catalytique (3) comprend un premier étage de conversion (32) pour la conversion catalytique et un deuxième étage de conversion (33) pour la conversion catalytique qui, quant au processus, est situé en aval du premier étage de conversion (32), et que la conversion catalytique (3) dans le premier étage de conversion (32) et le deuxième étage de conversion (33) se déroule à une température différente de sorte que l'équilibre chimique dans le premier étage de conversion (32) est différent de l'équilibre chimique dans le deuxième étage de conversion (33).

3. Procédé selon la revendication 2, **caractérisé par le fait qu'**un autre courant d'éduit (34) comprenant du monoxyde de carbone est amené à l'ensemble de récupération (3a), en particulier à la conversion catalytique (3) pour une conversion au moins partielle en hydrogène et en dioxyde de carbone par réaction avec de la vapeur d'eau, de préférence que le courant d'éduit (4) est situé, quant au processus, en aval de la conversion catalytique (3) du premier étage de conversion (32) et est situé, quant au processus, en amont du deuxième étage de conversion (33), en particulier que ledit autre courant d'éduit (34) est amené au premier étage de conversion (32).

4. Procédé selon l'une quelconque des revendications 1 à 3, **caractérisé par le fait qu'**au moins 80 %, de préférence au moins 85 %, en particulier au moins 90 %, de la proportion molaire de monoxyde de carbone du courant d'éduit (4), en particulier également de l'autre courant d'éduit (34), sont convertis dans la conversion catalytique (3), par réaction avec de la vapeur d'eau, en hydrogène et en dioxyde de carbone.

5. Procédé selon l'une quelconque des revendications 1 à 4, **caractérisé par le fait que** la conversion catalytique (3) comprend un étage de conversion catalytique à basse température (31) dans lequel se déroule pour l'essentiel exclusivement une réaction du gaz à l'eau à basse température, de préférence à moins de 300 °C, en particulier que le deuxième étage de conversion (33) est l'étage de conversion catalytique à basse température (31).

6. Procédé selon l'une quelconque des revendications 1 à 5, **caractérisé par le fait que** la conversion catalytique (3) comprend un étage de conversion catalytique à haute température (30) dans lequel se déroule pour l'essentiel exclusivement une réaction du gaz à l'eau à haute température, de préférence à au moins 300 °C, en particulier que le premier étage de conversion (32) est l'étage de conversion catalytique à haute température (30).

7. Procédé selon l'une quelconque des revendications 1 à 6, **caractérisé par le fait que**, quant au processus en amont de la conversion catalytique (3), un courant de gaz de synthèse (18) comprenant de l'hydrogène et des oxydes de carbone est amené à l'ensemble de réacteur de méthanol (6) pour une conversion au moins partielle en méthanol (1) de sorte que les oxydes de carbone qui n'ont pas réagi proviennent du courant de gaz de synthèse (18), de préférence que l'ensemble de réacteur de méthanol (6) comprend un étage de condensation (21) pour séparer le méthanol (1) et pour obtenir le courant de gaz résiduel (5).

8. Procédé selon la revendication 7, **caractérisé par le fait que** ledit ensemble de réacteur de méthanol (6) présente un premier étage de réacteur (20) et un deuxième étage de réacteur (26) pour la synthèse de méthanol (1), que le courant de gaz de synthèse (18) est amené au premier étage de réacteur (20) et qu'un autre courant de gaz résiduel (24) comprenant des oxydes de carbone qui n'ont pas réagi et provenant du premier étage de réacteur (20) est amené au deuxième étage de réacteur (26), de préférence que l'étage de condensation (21) est situé, quant au processus, entre le premier étage de réacteur (20) et le deuxième étage de réacteur (26) et que le courant de gaz résiduel (5) et l'autre courant de gaz résiduel (24) sont obtenus à partir de l'étage de condensation (21).

9. Procédé selon la revendication 7 ou 8, **caractérisé par le fait qu'**un courant porteur d'énergie (35) contenant du carbone est amené à un ensemble de réacteur de gaz de synthèse (36) pour obtenir le courant de gaz de synthèse (18), de préférence que ledit autre courant d'éduit (34) est un autre courant de gaz de synthèse (44) comprenant de l'hydrogène et des oxydes de carbone et obtenu à partir de l'ensemble de réacteur de gaz de synthèse (36), de préférence qu'un courant contenant de l'oxygène (40) est amené à l'ensemble de réacteur de gaz de synthèse (36) et que, dans un réacteur (38) de l'ensemble de réacteur de gaz de synthèse (36), on obtient du gaz de synthèse pour le courant de gaz de synthèse (18), en particulier également pour ledit autre courant de gaz de synthèse (44), par une oxydation partielle catalytique au moyen du courant contenant de l'oxygène (40).

10. Procédé selon la revendication 9, **caractérisé par le fait que** l'ensemble de réacteur de gaz de synthèse (36) présente une utilisation de la chaleur perdue (41) à plusieurs étages pour la récupération de la chaleur provenant de la production du gaz de synthèse, qui, quant au processus, est située en aval du réacteur (38), et que le courant de gaz de synthèse et l'autre courant de gaz de synthèse (44) sont obtenus après respectivement différents étages de l'utilisation de la chaleur perdue (41), de préférence que ledit autre courant de gaz de synthèse (44) est dérivé, quant au processus, en amont du courant de gaz de synthèse (18).

11. Procédé selon l'une quelconque des revendications 1 à 10, **caractérisé par le fait que** le courant d'azote (13) est obtenu à partir d'une séparation d'air (45) pour obtenir de l'oxygène moléculaire à partir de l'air ambiant, de préférence que le courant contenant de l'oxygène (40) est un courant d'oxygène obtenu à partir de la séparation d'air (45) et se composant en particulier pour l'essentiel d'oxygène.

12. Procédé selon l'une quelconque des revendications 1 à 11, **caractérisé par le fait que** le gaz de purge (12) comprenant du dioxyde de carbone est obtenu à partir de la récupération d'hydrogène (8), de préférence que dans la récupération d'hydrogène (8) est éliminée pour l'essentiel la totalité du dioxyde de carbone du courant converti (7) de sorte que le premier courant contenant de l'hydrogène (10) et/ou le deuxième courant contenant de l'hydrogène (11) est pour l'essentiel exempt de dioxyde de carbone.

13. Procédé selon la revendication 12, **caractérisé par le fait que** la récupération d'hydrogène (8) comprend un lavage de dioxyde de carbone (46) pour éliminer par lavage au moins en partie le dioxyde de carbone, au moyen d'un détergent, dudit courant converti (7), de préférence que le gaz de purge (12) est obtenu au moins en partie à partir du lavage de dioxyde de carbone (46), en particulier que le deuxième courant contenant de l'hydrogène (11) est obtenu à partir du lavage de dioxyde de carbone (46) en éliminant par lavage le dioxyde de carbone du courant converti (7).

14. Procédé selon l'une quelconque des revendications 1 à 13, **caractérisé par le fait que** la récupération d'hydrogène (8) comprend une installation d'adsorption à variation de pression (9) destinée à obtenir le premier courant contenant de l'hydrogène (10), de préférence également à obtenir le deuxième courant contenant de l'hydrogène (11), et qui, en particulier, quant au processus, est montée en aval du lavage de dioxyde de carbone (46), de préférence encore que le premier courant contenant de l'hydrogène (10) se compose pour l'essentiel d'hydrogène et est pour l'essentiel exempt d'oxyde de carbone, en outre en particulier que le deuxième courant contenant de l'hydrogène (11) se compose pour l'essentiel d'hydrogène et est pour l'essentiel exempt d'oxyde de carbone.

15. Installation de production combinée de méthanol (1) et d'ammoniac (2), comprenant un ensemble de récupération (3a) auquel est amené un courant d'éduit (4) comprenant du monoxyde de carbone et à partir duquel sont obtenus un premier courant contenant de l'hydrogène (10) et un deuxième courant contenant de l'hydrogène (11), ayant chacun une fraction molaire d'hydrogène accrue par rapport au courant d'éduit (4), dans lequel ledit ensemble de récupération présente une conversion catalytique (3) dans laquelle du monoxyde de carbone d'au moins un courant contenant du monoxyde de carbone (4a) est converti, au moins en partie par réaction avec de la vapeur d'eau, en hydrogène et en dioxyde de carbone de sorte que l'on obtienne un courant converti (7) comprenant de l'hydrogène et du dioxyde de carbone, dans lequel ledit ensemble de récupération (3a) présente une récupération d'hydrogène (8) à laquelle est amené, au moins en partie, le courant converti (7) et à partir de laquelle sont obtenus le premier courant contenant de l'hydrogène (11) et le deuxième courant contenant de l'hydrogène (11), avec un ensemble de réacteur d'ammoniac (14) auquel sont amenés un courant d'azote (13) et au moins en partie le premier courant contenant de l'hydrogène (10) pour les convertir au moins en partie en ammoniac (2), et avec un ensemble de réacteur de méthanol (6) auquel est amené au moins en partie le deuxième courant contenant de l'hydrogène (11) pour le convertir au moins en partie en méthanol (1), **caractérisé par le fait que** le courant d'éduit (4) comprend un courant de gaz résiduel (5) comprenant des oxydes de carbone issus de l'ensemble de réacteur de méthanol (6), qui n'ont pas réagi.
